(19) Europäisches Patentamt

European Patent Office

Office européen des brevets

(11) Numéro de publication: **0 285 730 B1**

(12) **FASCICULE DE BREVET EUROPEEN**

(45) Date de publication de fascicule du brevet: **01.07.92**  (51) Int. Cl.⁵: **A61K 31/415**

(21) Numéro de dépôt: **87400769.3**

(22) Date de dépôt: **07.04.87**

Le dossier contient des informations techniques présentées postérieurement au dépôt de la demande et ne figurant pas dans le présent fascicule.

(54) **Application du butyl-4-diphényl-1,2-pyrazolidine-dione-3,5 comme agent antiviral contre le SIDA.**

(43) Date de publication de la demande:
**12.10.88 Bulletin 88/41**

(45) Mention de la délivrance du brevet:
**01.07.92 Bulletin 92/27**

(84) Etats contractants désignés:
**BE CH DE GB IT LI LU NL SE**

(56) Documents cités:

MARTINDALE, The Extra Pharmacopoeia,
28ième Edition, pp. 273-275

Ouest Médical, vol. 29, no. 15-16; 1976, pp.
1079-1082

Gazette Médicale de France, vol. 86, no. 31,
1979, pp. 3545-3546

Le concours médical, vol. 101, no. 35, 1979,
pp. 5309-5319

Journal Neurology, vol. 229, no. 2, 1983, pp.
125-127

(73) Titulaire: **Miesch, Jean-Olivier**
**2,Place Jeanne d'Arc**
**F-78120 Rambouillet(FR)**

(72) Inventeur: **Miesch, Jean-Olivier**
**2,Place Jeanne d'Arc**
**F-78120 Rambouillet(FR)**

(74) Mandataire: **Rinuy, Santarelli**
**14, avenue de la Grande Armée**
**F-75017 Paris(FR)**

CHEMICAL ABSTRACTS, vol. 70, 1969, page 184, réf. no. 104872x Columbus, Ohio, US; A.D. INGLOT: "Comparison of the antiviral activity in vitro of some nonsterioidal antiinflammatory drugs"

CHEMICAL ABSTRACTS, vol. 77, 1972, page 112, réf. no. 14830x Columbus, Ohio, US; D. OLKOWSKA et al.: "Effect of some antipyretic drugs on Herpes simplex virus infection in tissue culture"

ARZNEIMITTELFORSCHUNG;(Drug Res.), vol. 25, no. 6, 1975, pages 878-879 A.S.SARATIKOV et al.: "Production of species-unspecific antiviral inhibitors in cell-cultures and in chicken embryos under the influence of Pyrazolon derivatives"

CHEMICAL ABSTRACTS, vol. 77, 1972, page 11, réf. no. 135040y; Columbus, Ohio, US A.S. SARATIKOV et al.: "Action of some pyrazolone derivatives on parameters of the mitotic cycle in a cell culture in vitro."

CHEMICAL ABSTRACTS, vol. 78, 1973, page 70, réf. no. 119649u; Columbus, Ohio, US A.S. SARATIKOV et al.: "Antiviral effect of some pyrazolone derivatives in vitro cell culture."

CHEMICAL ABSTRACTS, vol. 99, 1983, pages 597-598, réf.no. 192988s; Columbus, Ohio, US R.L. FORTI et al.: "A functional cycloosygenase enzyme is not required for mediation of the pleiotropic effects of human alpha or beta interferon."

CHEMICAL ABSTRACTS, vol. 101, 1984, page 434, réf. no. 88258c; Columbus, Ohio, US P.K. MUKHERJEE et al.: "Reversible restriction of vesicular stomatitis virus in permissive cells treated with inhibitors of prostaglandin biosynthesis."

CHEMICAL ABSTRACTS, vol. 81, 1974, page 92, réf. no. 21545u; Columbus, Ohio, US T. SKWAREDK et al.: "Effect of new thiosemicarbazone derivatives and some antiinflammatory drugs on the growth of influenza viruses A2 and B."

## Description

La présente invention a pour objet une seconde application thérapeutique d'un médicament connu.

Le brevet Français 983.378 de GEIGY a mentionné que le butyl-4-diphényl-1-2-pyrazolidine-dione-3,5, plus connu sous la marque de "phényl butazone", est un médicament utile comme analgésique et antipyrétique.

Ce produit, qui appartient à la classe des anti-inflammatoires non stéroïdiens a connu un très grand succès à l'échelon mondial, mais la crainte d'effets secondaires indésirables a conduit le ministère chargé de la Santé Publique à en limiter l'emploi à certaines affections rhumatologiques et à retirer du marché les spécialités présentées sous forme injectable.

Il a maintenant été découvert selon la présente invention que ce même produit chimique, le butyl-4-diphényl-1-2-pyrazolidinedione-3,5 est un agent antiviral remarquable contre le sida, maladie due à un virus dont le noyau est un ARN, pouvant aller jusqu'à une action totale avec "restitutio ad integrum".

Ce produit s'est révélé actif dans cette nouvelle application thérapeutique contre le sida.

Dans la nouvelle application selon l'invention, ledit produit actif peut être appliqué sous forme de suppositoires, de comprimés et d'injections à une dose de l'ordre de 10mg. par Kg de poids du corps du patient et par jour, soit à une dose fort éloignée de la dose léthale qui est de 4 g. par jour.

La lenteur du métabolisme dudit produit, dont l'affinité pour les protéines plasmatiques est particulièrement élevées et l'excrétion très lente du produit (l'hémicrèse ou demi-vieplasmatique du produit étant de l'ordre de 72 heures, ce qui est très long) permettent d'obtenir un effet prolongé du médicament, même après absorption par l'organisme d'une dose unique.

Le produit présentant une action sodorétentrice, une activité agressive vis-à-vis de la muqueuse gastrique en cas de traitement prolongé et une action agressive au niveau des cellules rénales, hépatiques et sangui-formatrices, il convient d'observer un certain nombre de précautions, lors de son application contre le SIDA.

Il convient, en cas d'association avec les anti-vitamines K de surveiller fréquemment la coagulabilité pour adapter la posologie à la présence d'anticoagulant; lors du traitement prolongé, il est souhaitable d'effectuer des bilans hépatiques et rénaux; en cas d'apparition d'oedèmes, il est recommandé d'instituer un régime alimentaire désodé strict en substituant éventuellement un sel de régime comme le chlorure de magnésium; enfin la prudence s'impose pour le traitement d'un sujet âgé ou hypertendu et si des troubles digestifs apparaissent au cours d'un traitement par administration orale, il convient de lui substituer un traitement par voie rectale.

Sans vouloir être lié par une explication quelconque du traitement selon l'invention, on peut supposer que, dans le cas de cette affection virale à virus de noyau ARN, les radicaux phényl-NH du produit actif réagissent avec la forme énolique de l'uracile qui est le dérivé de la pyrimidine rentrant avec la ribose et un phosphate dans la constitution du noyau de l'acide ribonucléïque pour le détruire.

## Revendications

1. Application du butyl-4-diphényl-1,2-pyrazolidine-dione-3,5 pour obtenir un médicament destiné à être utilisé contre le SIDA.

## Claims

1. Aplication of the butyl-4-diphenyl-1,2-pyrazolidine-dione-3,5 to obtain a medicine to use it against AIDS.

## Patentansprüche

1. Anwendung von 4-Butyl-1,2-diphenyl-pyrazolidin-3,5-dion zum Erhalten eines Arzneimittels gegen AIDS.